(19) Europäisches Patentamt
European Patent Office
Office européen des brevets

(11) **EP 2 750 648 B1**

(12) **EUROPÄISCHE PATENTSCHRIFT**

(45) Veröffentlichungstag und Bekanntmachung des Hinweises auf die Patenterteilung:
**01.03.2017 Patentblatt 2017/09**

(21) Anmeldenummer: **12745694.5**

(22) Anmeldetag: **09.08.2012**

(51) Int Cl.:
*A61F 13/495* (2006.01)    *A61F 13/496* (2006.01)
*A61F 13/49* (2006.01)

(86) Internationale Anmeldenummer:
**PCT/EP2012/065584**

(87) Internationale Veröffentlichungsnummer:
**WO 2013/029951 (07.03.2013 Gazette 2013/10)**

(54) **INKONTINENZARTIKEL IN HÖSCHENFORM**

PANTY-TYPE INCONTINENCE ARTICLE

ARTICLE POUR L'INCONTINENCE SOUS FORME DE CULOTTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.09.2011 DE 102011081984**

(43) Veröffentlichungstag der Anmeldung:
**09.07.2014 Patentblatt 2014/28**

(73) Patentinhaber: **Paul Hartmann AG**
**89522 Heidenheim (DE)**

(72) Erfinder: **MALOWANIEC, Krzysztof D.**
**89522 Heidenheim (DE)**

(74) Vertreter: **DREISS Patentanwälte PartG mbB**
**Friedrichstrasse 6**
**70174 Stuttgart (DE)**

(56) Entgegenhaltungen:
**DE-A1-102007 055 524    US-A- 5 558 660**

**Beschreibung**

Die Erfindung betrifft einen Inkontinenzartikel in Höschenform für die Aufnahme von Körperausscheidungen mit einem vorderen Bauchabschnitt und einem hinteren Rückenabschnitt, die in einer Längsrichtung des Inkontinenzartikels voneinander separat und beabstandet sind, und mit einem einen Absorptionskörper aufweisenden Schrittabschnitt, der sich in der Längsrichtung zwischen dem Bauchabschnitt und dem Rückenabschnitt erstreckt und an den Bauchabschnitt und an den Rückenabschnitt unlösbar angefügt ist, wobei der Bauchabschnitt und der Rückenabschnitt zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind und wobei sowohl der Schrittabschnitt als auch der Bauchabschnitt und der Rückenabschnitt Beinöffnungen des Inkontinenzartikels begrenzen, wobei in einem hüftseitigen Bereich des Bauchabschnitts und des Rückenabschnitts erste Elastifizierungsmittel vorgesehen sind, die sich in einem Abstand voneinander und parallel zueinander in Quer- oder Hüftumfangsrichtung erstrecken und so den Bauchabschnitt und den Rückenabschnitt in dem genannten hüftseitigen Bereich über dessen gesamte flächenhafte Erstreckung durchgehend elastifizieren, wobei in einem schrittseitigen und den Beinöffnungen zugewandten Bereich des Rückenabschnitts und vorzugsweise auch des Bauchabschnitts zweite Elastifizierungsmittel vorgesehen sind, die sich ausgehend von den beiden Seitennahtbereichen in Richtung auf eine Längsmittelachse des Inkontinenzartikels erstrecken, wobei die zweiten Elastifizierungsmittel im Überlappungsbereich des Absorptionskörpers mit dem Bauchabschnitt und mit dem Rückenabschnitt entweder nicht vorhanden oder ihrer elastifizierenden Wirkung benommen sind.

Ein derartiger dreikomponentiger Inkontinenzartikel ist beispielsweise bekannt aus DE 10 2007 055 524 A1 der Anmelderin.

Aus US 5,558,660 ist eine öffen- und schließbare Windel vorbekannt, bei der im Bereich des Längsendes des Absorptionskörpers ein dreidimensionales zeltartiges Taschencuff-Element auf der körperzugewandten Seite des Absorptionskörpers vorgesehen und an eine körperzugewandte Seite des Artikels angefügt ist. Es verfügt über einen elastifizierten schrittzugewandten Rand und kooperiert mit in Längsrichtung verlaufenden cuff-Elementen, so dass es sich von dem darunter liegenden Schrittabschnitt erheben und eine in Längsrichtung öffnende Tasche für Körperausscheidungen bilden kann.

Bei diesem spezifischen Produktkonzept kann nach dem Anfügen des in Längsrichtung erstreckten Schrittabschnitts an den im Wesentlichen in Quer- oder Hüftumfangsrichtung erstreckten Bauchabschnitt und den entsprechend erstreckten Rückenabschnitt im eben ausgebreiteten Zustand dieser drei Komponenten eine H-förmige Grundstruktur des Inkontinenzartikels realisiert werden. Der Inkontinenzartikel ist dann modular aus den Komponenten Schrittabschnitt, Bauchabschnitt und Rückenabschnitt gebildet. Diese Komponenten werden vorteilhafterweise zunächst über den Schrittabschnitt miteinander verbunden, und vorzugsweise danach wird in beidseitigen Seitennahtbereichen der Bauchabschnitt mit dem Rückenabschnitt verbunden. Hierbei handelt es sich um eine herstellerseitige Verbindung, durch welche die Höschenform erhalten wird. Diese Verbindung ist typischerweise unlösbar. Die Höschenform kann aber auch insbesondere entlang einer Sollbruchlinie, die insbesondere im Seitennahtbereich verlaufen kann, auftrennbar sein, beispielsweise zum Abnehmen eines gebrauchten Inkontinenzartikels bei einer pflegebedürftigen Person.

Inkontinenzartikel in Höschenform unterscheiden sich prinzipiell von öffenbaren und schließbaren Inkontinenzartikeln in üblicher Windelform dadurch, dass in der Regel der Hüftumfang vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra-Fäden bezeichnet, in vorgedehntem Zustand (Stretch-Bond-Verfahren) mit Chassismaterialien des Inkontinenzartikels verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen. Der Inkontinenzartikel bzw. die elastifizierten Chassismaterialien des Inkontinenzartikels lassen sich dann wieder elastisch dehnen, wenn der Inkontinenzartikel wie ein Höschen an den Benutzer angelegt wird. Inkontinenzartikel in Höschenform mit derart elastifizierten Chassismaterialien sind mehrfach bekannt geworden und beispielsweise auch in der vorerwähnten DE 10 2007 055 524 A1 behandelt.

Der vorliegenden Erfindung liegt die Aufgabe zugrunde, die Verwendbarkeit eines Inkontinenzartikels der eingangs genannten Art im Hinblick auf kritische Betriebsbedingungen, wie sie insbesondere bei niedrigviskosen bzw. halbfesten Körperausscheidungen auftreten, noch weitergehend zu verbessern.

Diese Aufgabe wird bei einem Inkontinenzartikel der genannten Art erfindungsgemäß durch die Merkmale des Anspruchs 1 gelöst. Es wird vorgeschlagen, dass ein hinterer dem Rückenabschnitt zugewandter Endbereich des Absorptionskörpers in Längsrichtung und in Querrichtung von einem Flachmaterialabschnitt überfangen ist, der auf der körperzugewandten Seite des Schrittabschnitts vorgesehen ist und an den Schrittabschnitt unlösbar angefügt ist, und dass der Flachmaterialabschnitt einen hüftzugewandten im wesentlichen in Querrichtung verlaufenden Rand und einen schrittzugewandten im wesentlichen in Querrichtung verlaufenden Rand und zwei Seitenränder aufweist und dass der hüftzugewandte Rand in Längsrichtung einen Abstand vom benachbarten Längsende des Rückenabschnitts aufweist, und dass der Flachmaterialabschnitt im Bereich seines schrittzugewandten Rands oberhalb des Absorptionskörpers nicht gegen den Schrittabschnitt fixiert ist, so dass er sich von dem darunter liegenden Schrittabschnitt erheben und eine in Längsrichtung öffnende Tasche für Körperausscheidungen bilden kann, und dass der schrittzugewandte Rand des Flachmaterialabschnitts elastisch oder elastifiziert ausgebildet ist.

Es wird also erfindungsgemäß vorgeschlagen, dass im nach hinten gewandten Bereich des Schrittabschnitts eine von dem Flachmaterialabschnitt gebildete Barriere geschaffen wird, welche wirksam verhindert, dass Körperausscheidungen in Richtung auf die Hüftöffnung und zu den Seiten hin wandern. Gerade bei den hier in Rede stehenden aus den drei Hauptkomponenten gebildeten Inkontinenzartikeln werden nämlich zur Bildung des Rückenabschnitts typischerweise stark atmungsaktive Vliesstoffe verwendet, die demgemäß dünn ausgebildet sind; außerdem wird bei den Materialien des Rückenabschnitts typischerweise auf flüssigkeitsundurchlässige Folienschichten verzichtet. Insofern stellen wandernde niedrigviskose oder halbfeste Körperausscheidungen und insbesondere flüssiger Stuhl ein großes Betriebsproblem bei derartigen Inkontinenzartikeln dar. Durch die erfindungsgemäße Maßnahme der Schaffung einer Barriere durch den sich von der körperzugewandten Seite erhebenden Materialabschnitt, der eine Tasche bildet, kann diesbezüglich eine wesentliche Verbesserung des Betriebsverhaltens des Inkontinenzartikels erreicht werden. Es wurde zudem festgestellt, dass die Weglassung oder Deaktivierung der zweiten Elastifizierungsmittel im schrittseitigen Bereich des Rückenabschnitts unterhalb des Absorptionskörpers im Zusammenwirken mit der elastischen oder elastifizierten Ausbildung des schrittzugewandten Rands des Flachmaterialabschnitts die Taschenbildung, also das Abheben des Flachmaterialabschnitts von der körperzugewandten Oberseite des Schrittabschnitts begünstigt. Hierdurch wird aber die Barrierewirkung dieser Maßnahme noch weitergehend verbessert. Hieraus ergibt sich der weitere Vorteil, dass an der atmungsaktiven und damit hautfreundlichen Ausbildung des Rückenabschnitts, auch im unmittelbaren Bereich des Schrittabschnitts festgehalten werden kann.

Des weiteren wird vorgeschlagen, dass der Flachmaterialabschnitt mit seinem hüftzugewandten im wesentlichen in Querrichtung verlaufenden Rand und mit seinen beiden Seitenrändern bis wenigstens nahezu an die Ränder des Schrittabschnitts heranreicht, also nicht über die Peripherie des Schrittabschnitts übersteht. Diese weitere Maßnahme bringt den Vorteil mit sich, dass der typischerweise mit einer geringen oder gegen Null gehenden Flüssigkeitsdurchlässigkeit, in jedem Fall aber mit einer Mediendichtigkeit ausgebildete Flachmaterialabschnitt die Atmungsaktivität des Rückenabschnitts jedenfalls außerhalb des Überlappungsbereichs mit dem Schrittabschnitt nicht negativ beeinflusst. Außerdem eröffnet dies die Möglichkeit, dass der genannte Flachmaterialabschnitt im Zuge der Herstellung und Fertigstellung des Schrittabschnitts am Schrittabschnitt konfiguriert und befestigt werden kann. Die Ausbildung, Anordnung und Fixierung des Flachmaterialabschnitts wird also der modularen Herstellung des Schrittabschnitts als einer der drei Komponenten des Inkontinenzartikels zugeordnet.

Des weiteren wird vorgeschlagen, dass der Flachmaterialabschnitt entlang seines hüftzugewandten im wesentlichen in Querrichtung verlaufenden Rands und entlang seiner beiden Seitenränder kontinuierlich oder diskontinuierlich gegen die körperzugewandte Seite des Schrittabschnitts fixiert ist. Die Fixierung kann beispielsweise mittels Kleber oder durch Ultraschallschweißverbindungen oder in anderer Weise realisiert werden. Es können hierfür beispielsweise linienförmige oder streifenförmige Fügebereiche, die durchgehend oder unterbrochen ausgebildet sind, und insbesondere punktförmige Fügestellen eingesetzt werden.

Weiter erweist es sich als vorteilhaft, wenn der schrittzugewandte Rand des Materialabschnitts über seine gesamte Länge elastisch oder elastifiziert ausgebildet ist. Die elastische oder elastifizierte Ausbildung erstreckt sich dann gewissermaßen vom linken Seitenrand zum rechten Seitenrand des Flachmaterialabschnitts. Es wäre indessen auch denkbar, dass nur ein Teilbereich, insbesondere der Teilbereich oberhalb des Absorptionskörpers elastisch oder elastifiziert ausgebildet ist.

Nach einer Weiterbildung der Erfindung erstreckt sich der schrittzugewandte Rand des Flachmaterialabschnitts in Längsrichtung über den schrittzugewandten Rand des Rückenabschnitts hinaus. Nach einer alternativen Ausführungsform wäre es aber auch denkbar, den Flachmaterialabschnitt in der Längsrichtung betrachtet kürzer auszubilden. Solchenfalls würde sich der schrittzugewandte Rand vorzugsweise lediglich über den hüftzugewandten Rand des Absorptionskörpers hinaus erstrecken, nicht aber über den schrittzugewandten Rand des Rückenabschnitts. Vorzugsweise würde sich der schrittzugewandte Rand des Flachmaterialabschnitts solchenfalls gleichwohl bis in den schrittseitigen und den Beinöffnungen zugewandten Bereich des Rückenabschnitts erstrecken.

Der hüftzugewandte Rand des Flachmaterialabschnitts weist in Längsrichtung erfindungsgemäß einen Abstand vom benachbarten Längsende des Rückenabschnitts auf. Dieser Abstand beträgt vorzugsweise 4-20 cm, insbesondere 6-15 cm.

Nach einem weiteren Erfindungsgedanken von besonderer Bedeutung wird vorgeschlagen, dass der Absorptionskörper in seinem hinteren dem Rückenabschnitt zugewandten Endbereich wenigstens eine ungefähr in Längsrichtung erstreckte Flexibilitäts- oder Sollbiegeachse aufweist, was die Bildung der in Längsrichtung öffnenden Tasche begünstigt. Auf diese Weise lässt sich eine im Querschnitt betrachtet nach oben konkav öffnende, also schalenförmige Gestalt des Absorptionskörpers erreichen, die wiederum die Taschenbildung durch den Flachmaterialabschnitt und dessen Abheben von der körperzugewandten Seite des Schrittabschnitts fördert. Die erwähnte Flexibilitäts- oder Sollbiegeachse kann im einfachsten Fall durch eine oder mehrere, vorzugsweise zwei Präge- oder Verdichtungslinien in dem Material des Absorptionskörpers gebildet werden. Es wäre aber auch denkbar, dass die Flexibilitäts- oder Sollbiegeachse durch ein geringeres Flächengewicht der Materialien des Absorptionskörpers entlang der betreffenden Flexibilitäts- oder Sollbiegeachse gebildet ist. Weiter erweist es sich als vorteilhaft, wenn die Flexibilitäts- oder Sollbiegeachse sich bis zum hüftseitigen Ende des Absorptionskörpers erstreckt und dort ausmündet.

Es erweist sich weiter als vorteilhaft, wenn die Erstreckung des Flachmaterialabschnitts von seinem schrittzugewandten Rand in Längsrichtung nach hinten bis zu einer Fixierung im Bereich des hüftzugewandten im wesentlichen in Querrichtung verlaufenden Rands wenigstens 2 cm, insbesondere wenigstens 3 cm, insbesondere wenigstens 4 cm, insbesondere wenigstens 5 cm und weiter insbesondere höchstens 15 cm, insbesondere höchstens 12 cm, insbesondere höchstens 10 cm, insbesondere höchstens 8 cm aufweist.

In bestimmten Situationen kann es sich als vorteilhaft erweisen, wenn zusätzlich im Bereich des bauchseitigen Endes des Absorptionskörpers eine entsprechende Barriere vorgesehen ist. Insofern wird vorgeschlagen, dass ein vorderer dem Bauchabschnitt zugewandter Endbereich des Absorptionskörpers in Längsrichtung und in Querrichtung von einem Flachmaterialabschnitt überfangen ist, der auf der körperzugewandten Seite des Schrittabschnitts vorgesehen ist und an den Schrittabschnitt und/oder an den Bauchabschnitt unlösbar angefügt ist, und dass der Flachmaterialabschnitt einen hüftzugewandten im wesentlichen in Querrichtung verlaufenden Rand und einen schrittzugewandten im wesentlichen in Querrichtung verlaufenden Rand und zwei Seitenränder aufweist und dass der hüftzugewandte Rand in Längsrichtung einen Abstand vom benachbarten Längsende des Bauchabschnitts aufweist, und dass der Flachmaterialabschnitt im Bereich seines schrittzugewandten Rands oberhalb des Absorptionskörpers nicht gegen den Schrittabschnitt fixiert ist, so dass er sich von dem darunter liegenden Schrittabschnitt erheben und eine in Längsrichtung öffnende Tasche für Körperausscheidungen bilden kann, und dass der schrittzugewandte Rand des Flachmaterialabschnitts elastisch oder elastifiziert ausgebildet ist.

Weiter erweist es sich als vorteilhaft, wenn die zweiten Elastifizierungsmittel, die sich, ausgehend von den beiden Seitennahtbereichen in Richtung auf eine Längsmittelachse des Inkontinenzartikels erstrecken, dabei bogenförmig verlaufen und sich in Richtung auf die Längsmittelachse auffächern, so dass ihr Abstand voneinander zunimmt. Vorzugsweise ist der Abstand der zweiten Elastifizierungsmittel voneinander maximal im Übergang zur Überlappung des Schrittabschnitts mit dem Rückenabschnitt bzw. Bauchabschnitt.

Hinsichtlich des Verlaufs und der Ausbildung der zweiten Elastifizierungsmittel, insbesondere hinsichtlich ihres Auffächerungsgrads und der durch sie erzeugten Rückstellkräfte bei flächenhafter Dehnung dieses schrittseitigen Bereichs des Rückenabschnitts und des Bauchabschnitts wird der Inhalt von DE 10 2007 055 524 A1 in die vorliegende Anmeldung einbezogen.

Weitere Merkmale, Einzelheiten und Vorteile der Erfindung ergeben sich aus den beigefügten Patentansprüchen und der zeichnerischen Darstellung und nachfolgenden Beschreibung einer bevorzugten Ausführungsform des erfindungsgemäßen Inkontinenzartikels. In der Zeichnung zeigt:

Figur 1 eine Draufsicht auf einen vorbekannten Inkontinenzartikel, wobei ein Bauchabschnitt, ein Rückenabschnitt und ein die beiden verbindender Schrittabschnitt des Inkontinenzartikels noch nicht zur Bildung einer Höschenform verbunden, sondern in flachgelegtem und ausgedehntem Zustand dargestellt sind;

Figur 2 eine ausschnittsweise Darstellung des Inkontinenzartikels nach Figur 1;

Figur 3 eine Draufsicht auf den Schrittabschnitt des Inkontinenzartikels nach Figur 1 wiederum im flachgelegten und ausgedehnten Zustand;

Figur 4 eine Schnittansicht (schematisch) entlang einer Quermittelachse des Schrittabschnitts mit Schnittebene IV-IV in Figur 3;

Figur 5 eine Figur 4 entsprechende Schnittansicht (schematisch) des Schrittabschnitts mit Schnittebene V-V in Figur 3, mit entfalteten und emporstehenden Barrieremitteln;

Figur 6 eine perspektivische Ansicht (schematisch) des an einen Benutzer angelegten Inkontinenzartikels nach Figur 1;

Figur 7 eine Figur 1 entsprechende Draufsicht des Inkontinenzartikels zur Verdeutlichung der Verbindung von Schrittabschnitt und Bauchabschnitt bzw. Rückenabschnitt;

Figur 8 verdeutlicht beispielhaft die Bestimmung von Rückstellkräften im Bauchabschnitt bzw. Rückenabschnitt des Inkontinenzartikels;

Figur 9 zeigt einen erfindungsgemäß ausgebildeten Inkontinenzartikel.

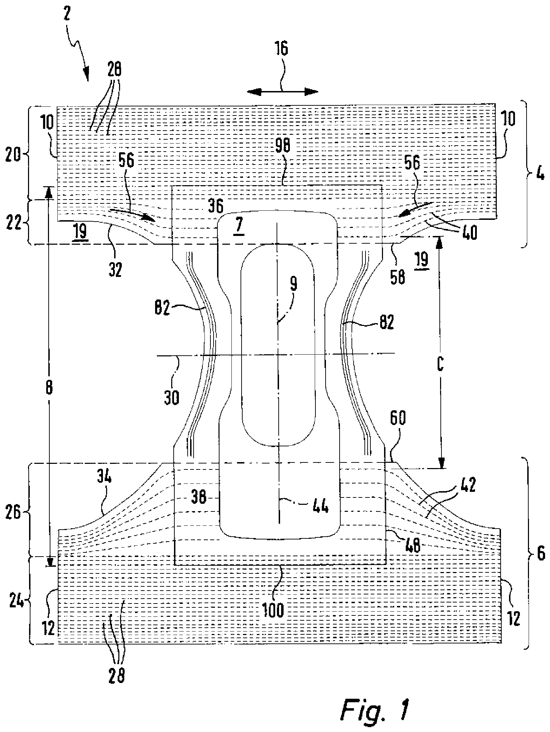

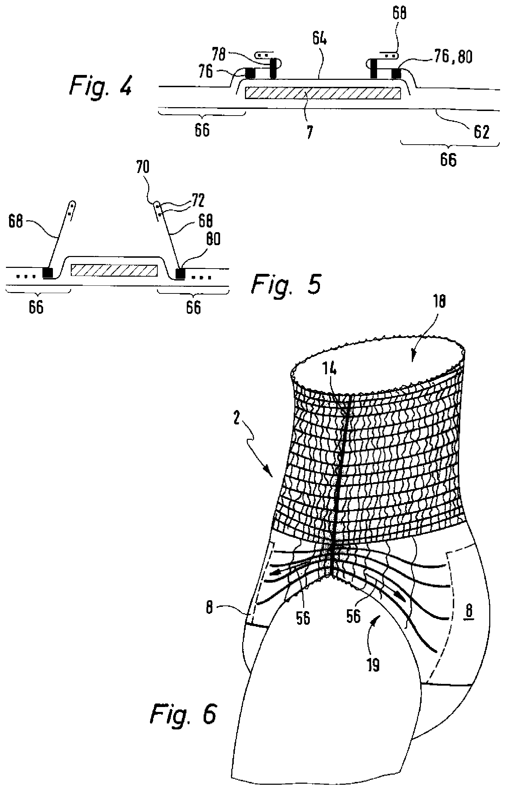

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 mit einem wesentlichen Flächenanteil des Bauchabschnitts 4 einerseits und des Rückenabschnitts 6 andererseits überlappt und im Überlappungsbereich herstellerseitig unlösbar verbunden ist. Wie aus Figur 1 ersichtlich, führt dies zu einer H-förmigen Grundstruktur des Inkontinenzartikels mit einer Längsrichtung 9. Die in Figur 1 dargestellten miteinander gefügten Bestandteile werden dann zur Bildung der in Figur 6 schematisch dargestellten Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 (Figur 6) ausgebildet werden. In diesem herstellerseitig gefertigten höschenförmigen Zustand des Inkontinenzartikels erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 und Beinöffnungen 19, durch welche hindurch der Benutzer den Inkontinenzartikel wie ein Höschen anlegt.

Der Bauchabschnitt 4 lässt sich in einen hüftseitigen Bereich 20 und in einen schrittseitigen und den Beinöffnungen zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen zugewandten Bereich 26.

In dem hüftseitigen Bereich 20 des Bauchabschnitts 4 und in dem hüftseitigen Bereich 24 des Rückenabschnitts 6 sind erste Elastifizierungsmittel 28 vorgesehen, bei denen es sich insbesondere um fadenförmige Elastifizierungsmittel, wie Lycra®-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28 erstrecken sich in Quer- oder Hüftumfangsrichtung 16 von einem Seitennahtbereich 14 zum anderen.

Der schrittseitige und den Beinöffnungen 19 zugewandte Abschnitt 22 des Bauchabschnitts 4 bzw. 26 des Rückenabschnitts 6 haben eine von der Quer- oder Hüftumfangsrichtung 16 abweichende in Richtung auf eine Quermittelachse 30 des Schrittabschnitts 8 zulaufende Randkontur 32 bzw. 34. Diese Randkontur 32, 34 ist auch in der Darstellung nach Figur 1 bogenförmig und daher zur Begrenzung der Beinöffnungen 19 geeignet. Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist. Je größer der Überlappungsbereich 36, 38, umso geringere Klebermengen bezogen auf die Fläche des Überlappungsbereiches können verwendet werden, was sich im Hinblick auf die Steifigkeit der Chassismaterialien als vorteilhaft auswirkt. Insbesondere kann ein nicht vollflächiger Kleberauftrag zur Verbindung der Komponenten verwendet werden.

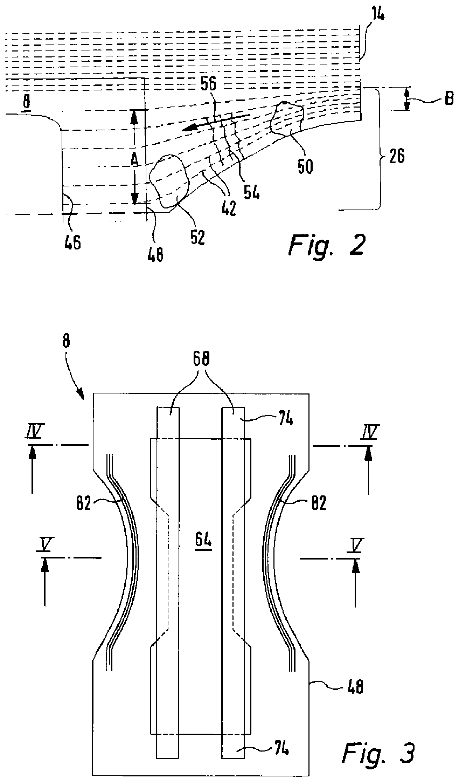

Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus den Figuren 1 und 2 zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt. Diese Auffächerung der zweiten Elastifizierungsmittel 40 bzw. 42 lässt sich anhand Figur 2 auch quantitativ näher bezeichnen. Beispielsweise haben die in Figur 2 dargestellten zweiten Elastifizierungsmittel 42 des Rückenabschnitts 6 in den Seitennahtbereichen 14 einen minimalen Abstand von 3 bis 8 mm voneinander (Abstand von unmittelbar nebeneinander liegenden Elastifizierungsmitteln) und an einem Absorptionskörperrand 46 oder einem Längsrand 48 des Schrittabschnitts 8 einen maximalen Abstand (Abstand von unmittelbar nebeneinander liegenden Elastifizierungsmitteln) von 7 bis 35 mm voneinander. Es lässt sich anhand der Figur 2 auch ein Auffächerungsgrad F wie folgt definieren:

$$F = (A-B)/B * 100\%.$$

Dieser Auffächerungsgrad kann vorteilhafterweise zwischen 50 und 900 %, insbesondere zwischen 100 und 700 % und weiter insbesondere zwischen 150 bis 550 % liegen. Er ist im Rückenabschnitt 6 vorteilhafterweise größer als im Bauchabschnitt 4. Dabei sind die Größen A und B definiert als der Abstand des in Längsrichtung 9 äußersten zweiten Elastifizierungsmittels 40, 42 von dem in Längsrichtung 9 innersten zweiten Elastifizierungsmittel 40, 42 (also nicht der Abstand von unmittelbar nebeneinander liegenden Elastifizierungsmitteln), und zwar A als der maximale Abstand, insbesondere am Längsrand 48 des Schrittabschnitts 8, und B als der minimale Abstand, insbesondere im Seitennahtbereich 14.

Sofern der Auffächerungsgrad bei den zweiten Elastifizierungsmitteln 40, 42 hinreichend groß gewählt wird, so lässt sich auf diese Weise eine abnehmende Rückstellkraft innerhalb des schrittseitigen und den Beinöffnungen 19 zugewandten Bereichs 22 beziehungsweise 26 in Richtung 56 auf den Schrittabschnitt 8 realisieren, sofern dafür Sorge getragen wird, dass in Folge des von der Hüft- oder Querrichtung 16 abgewandten bogenförmigen Verlaufs der zweiten Elastifizierungsmittel 40, 42 sich keine zu starke Erhöhung der Vorspannung in Folge des größeren Wegs dieser zweiten Elastifizierungsmittel 40, 42 ergibt. Betrachtet man dann ein näher am Seitennahtbereich 14 liegendes Gebiet 50 des betreffenden schrittseitigen Bereichs 22 beziehungsweise 26 mit einem näher am Schrittabschnitt 8 liegenden Gebiet 52, so ist die Rückstellkraft, die sich bei flächenhafter Dehnung des Gebiets 52 (Dehnung in Richtung der Elastifizierungsmittel 42) einstellt geringer als diejenige Rückstellkraft, die sich bei Dehnung des Gebiets 50 einstellt. Dies führt in vorteilhafter Weise weiter dazu, dass in Folge der geringeren elastischen Kräfte, welche im beispielhaft dargestellten Fall durch die zweiten Elastifizierungsmittel 40, 42 ausgeübt werden, die Chassismaterialien des Bauchabschnitts 4 und des Rückenabschnitts 6 weniger stark gerafft werden, so dass sich auch eine geringere Anzahl von Falten/Rüschen 54 ergibt, und zwar ausgehend vom jeweiligen Seitennahtbereich 14 in Richtung auf den Schrittabschnitt 8. Dadurch, dass die sich bei flächenhafter Dehnung des Bauchabschnitts in dem schrittseitigen und den Beinöffnungen zugewandten Bereich 22 des Bauchabschnitts 4 beziehungsweise 26 des Rückenabschnitts 6 sich einstellenden Rückstellkräfte in Richtung des Pfeils 56, also generell vom Seitennahtbereich 14 in Richtung auf den Schrittabschnitt 8 abnehmen, wird eine erhebliche Verbesserung des Tragekomforts erzielt, weil gerade in diesen Bereichen - wie erfindungsgemäß festgestellt wurde-sich elastisch dehnbare Materialien als besonders problematisch erweisen, weil diese Materialien der Physiognomie der menschlichen Körperform entsprechend in diesen Bereichen besonders auf Zug und Dehnung beansprucht werden. Durch eine erfindungsgemäß bewusst vorgesehene Verringerung dieser Rückstellkraft, also abnehmende Rückstellkraft in Richtung des Pfeils 56, das heißt in Richtung zunehmender Annäherung an den Schrittabschnitt 8, wird hier ein zuvor nicht realisierter Freiheitsgrad geschaffen, mit dem die eingangs geschilderten Probleme überwunden werden.

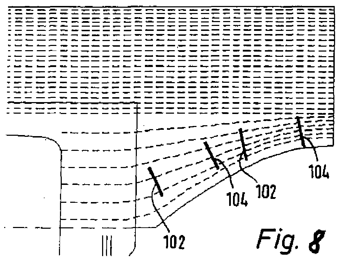

Wie eingangs ausgeführt, können Rückstellkräfte direkt am Chassis des Inkontinenzartikels bestimmt werden. Hierfür wird der betreffende Bereich des Bauchabschnitts 4 oder des Rückenabschnitts 6 zwischen zwei Klemmbacken 102, 104 (siehe Figur 8) von definierter, gleicher Klemmbackenbreite (b) eingespannt, und es werden dann Rückstellkräfte bei einer definierten, den Gebrauchszustand simulierenden Dehnung der zu messenden Bereiche um insbesondere 30% oder 50% oder 80% der Ausgangslänge (des Klemmbackenabstands im ungespannten Zustand) ermittelt. Die Klemmbacken 102, 104 werden dabei jeweils voneinander wegbewegt. Die Klemmbacken 102, 104 sollten möglichst viele, zumindest jedoch zwei nebeneinander angeordnete Elastifizierungsmittel 40, 42 des zu messenden Bereichs fixieren, und sie sollten im Wesentlichen rechtwinklig zum Verlauf der Elastifizierungsmittel orientiert sein, so dass die Dehnung zwischen den Klemmbacken 102, 104, also die Auseinanderbewegung der Klemmbacken 102, 104, im Wesentlichen in Richtung des Verlaufs der Elastifizierungsmittel erfolgt. Dies ist in Figur 8 verdeutlicht.

Bei der dargestellten bevorzugten Ausführungsform des Inkontinenzartikels 2 beträgt ein Abstand C des schrittzugewandten innersten zweiten Elastifizierungsmittels 40 des Bauchabschnitts 4 von dem entsprechenden schrittzugewandten innersten zweiten Elastifizierungsmittel 42 des Rückenabschnitts 6 zwischen 250 bis 420 mm je nach Konfektionsgröße des Inkontinenzartikels. Die zweiten Elastifizierungsmittel 40, 42 erstrecken sich im Wesentlichen bis zum schrittzugewandten Querrand 58, 60 des Bauchabschnitts 4 und des Rückenabschnitts 6. Der Abstand von Bauchabschnitt 4 und Rückenabschnitt 6 in Längsrichtung (9) voneinander beträgt 250 - 400 mm.

Der Abstand der innersten, schrittzugewandten zweiten Elastifizierungsmittel 40, 42 von der die Beinöffnungen begrenzenden Randkontur 32, 34 des schrittseitigen und den Beinöffnungen zugewandten Bereichs 22, 26 des Bauchabschnitts 4 und des Rückenabschnitts 6 beträgt vorzugsweise 2 - 40 mm, weiter vorzugsweise 3 - 30 mm, insbesondere vorzugsweise 4 - 15 mm.

Die Erstreckung des Bauchabschnitts 4 und des Rückenabschnitts 6 im Seitennahtbereich 14 in Längsrichtung 9 beträgt vorteilhafterweise zwischen 100 und 220 mm. Die maximale Erstreckung des Schrittabschnitts 8 in Querrichtung 16 beträgt vorteilhafterweise 200 bis 350 mm.

Der Schrittabschnitt 8 umfasst ein flüssigkeitsundurchlässiges Backsheet-Material 62, das insbesondere von einem atmungsaktiven, jedoch flüssigkeitsdichten Folienmaterial gebildet sein kann, und ein, vorzugsweise auf Vliesbasis beruhendes Topsheet-Material 64. Zwischen dem Backsheet-Material und dem Topsheet-Material ist wie aus den Figuren 4, 5 ersichtlich der Absorptionskörper 7 angeordnet. Im beispielhaft dargestellten Fall bildet das Backsheet-Material 62 in Querrichtung 16 einen Überhang 66 über den Absorptionskörper 7. Das Topsheet 64 überragt den Absorptionskörper 7 in Querrichtung nur verhältnismäßig geringfügig; jedoch ist beidseits des Absorptionskörpers 7 in Längsrichtung 9 verlaufend jeweils ein aufstehendes Barrieremittel 68 vorgesehen, welches typischerweise als aufstehendes Cuffelement oder Bündchenelement bezeichnet wird und vorzugsweise aus einem hydrophoben, insbesondere flüssigkeitsundurchlässigen Vliesstoffmaterial gebildet ist, welches sich in Querrichtung 16 vorzugsweise bis zu den seitlichen Längsrändern 48 des Schrittabschnitts 8 erstreckt. Die distalen Enden 70 der Barrieremittel 68 sind mit weiteren Elastifizierungsmitteln 72 versehen, welche die Barrieremittel 68 im Gebrauch des Inkontinenzartikels gegen die Hautoberfläche des Benutzers anheben, so wie dies in Figur 5 schematisch dargestellt ist. An ihren jeweiligen Längsendbereichen 74 sind die seitlichen Barrieremittel 68 über schematisch angedeutete Fixierungen 76, 78 auf das Topsheet 64 beziehungsweise auf sich selbst in einer C-förmig gefalteten Konfiguration festgelegt. Vorteilhaft und erwähnenswert ist hier, dass die in der Figur 4 jeweils innenliegende Fixierung 78 das Barrieremittel 68 auf sich selbst festlegt, und zwar in Querrichtung 16 innerhalb der äußeren Fixierung 76, welche eine in Längsrichtung 9 durchgehend erstreckte Cuffsockellinie 80 bildet. Die innere Fixierung 78 ist hingegen nur in den Längsendbereichen 74 der Barrieremittel 68 vorgesehen.

Es erweist sich hier als besonders vorteilhaft, wenn der erwähnte Überhang 66 des Backsheet-Materials 62 und/oder des Topsheet-Materials 64 über den Absorptionskörper 7 - in der Summe beidseits des Absorptionskörpers, also in der Summe links und rechts - wenigstens 25 % bezogen auf die größte Breite des Schrittabschnitts 8 beträgt. Auf diese Weise ist nämlich in Querrichtung 16 Raum für die Anordnung entlang der Beinöffnungen 19 erstreckter Beinelastifizierungsmittel 82. Es erweist sich nämlich als vorteilhaft, wenn die Beinelastifizierungsmittel 82 mit einem gewissen Abstand vom materialreichen und damit eher biegesteifen Absorptionskörper 7 verlaufen, um einerseits keine zusätzlichen Dehnungs- oder Verwindungskräfte auf den Absorptionskörper auszuüben, was dessen Absorptionsverhalten nachteilig beeinflussen könnte, und um andererseits einen vom Absorptionskörper weitgehend unbeeinflussten flüssigkeitsdichten Beinabschluss zu realisieren. Es erweist sich im dargestellten Fall als besonders vorteilhaft, dass diese Beinelastifizierungsmittel 82 in Längsrichtung 9 mit einem deutlichen Abstand von insbesondere wenigstens 10 mm, vorzugsweise wenigstens 20 mm vor den zweiten Elastifizierungsmitteln 40 und 42 des Bauchabschnitts 4 beziehungsweise des Rückenabschnitts 6 enden. Vorzugsweise enden diese Beinelastifizierungsmittel 82 in Längsrichtung 9 vor dem Bauchabschnitt 4 und dem Rückenabschnitt 6. Dies ist deshalb vorteilhaft und wesentlich, weil die Beinelastifizierungsmittel 82 solchenfalls das Spannungsverhalten des Bauchabschnitts 4 und des Rückenabschnitts 6 wenig oder gar nicht beeinflussen. Es wurde nämlich erkannt, dass es sich im Hinblick auf das erfindungsgemäß zu erreichende Ziel der Verbesserung des Tragekomforts gerade in dem schrittseitigen und den Beinöffnungen 19 zugewandten Bereich 22 und 26 des Bauchabschnitts 4 und des Rückenabschnitts 6 als negativ erweist, wenn dort die üblicherweise mit großer Vorspannung und entsprechend großer Rückstellkraft ausgebildeten Beinelastifizierungsmittel 82 zusätzlich verlaufen.

Wie aus Figur 1 ersichtlich ist, ist bei dem Schrittabschnitt 8 ein in Querrichtung verhältnismäßig großer Überhang 66 über den Absorptionskörper 7 realisiert, und zwar insbesondere auch an dem Bauchabschnitt 4 beziehungsweise dem Rückenabschnitt 6 zugewandten Bereichen des Schrittabschnitts 8. Hierdurch wird - worauf bereits hingewiesen wurde - ein verhältnismäßig großer Überlappungsbereich 36, 38 des Schrittabschnitts 8 mit dem Bauchabschnitt 4 und mit dem Rückenabschnitt 6 realisiert. Nach einer bevorzugten Ausführungsvariante umfasst der Überlappungsbereich 36 von Schrittabschnitt 8 mit dem Bauchabschnitt 4 wenigstens 12% der Fläche des Bauchabschnitts 4, und der Überlappungsbereich 38 von Schrittabschnitt 8 mit dem Rückenabschnitt 6 umfasst wenigstens 20% der Fläche des Rückenabschnitts 6. Dies erweist sich als vorteilhaft, da solchenfalls eine sichere Fixierung des Schrittabschnitts 8 an dem Bauchabschnitt 4 beziehungsweise an dem Rückenabschnitt 6 erreicht werden kann, und zwar auch, wenn kein vollflächiger Kleberauftrag verwendet wird. Es ist solchenfalls in vorteilhafter Weise hinreichend, wenn ein nur abschnittsweiser oder gerasterter Kleberauftrag zur Verbindung verwendet wird. Dies ist deshalb vorteilhaft, weil solchenfalls die miteinander gefügten Materialien nicht zu steif werden.

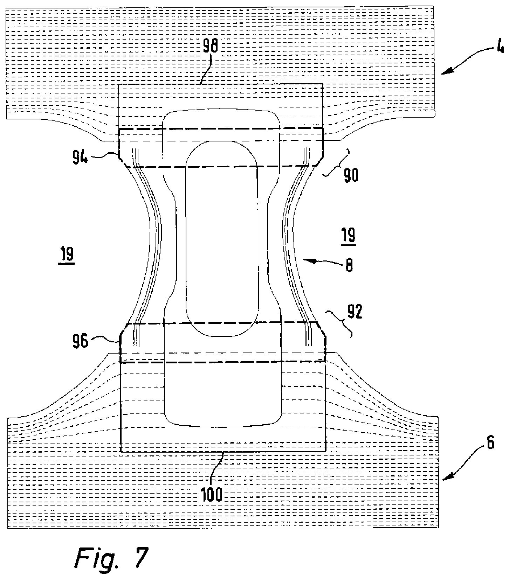

Anhand der Figur 7, die der Figur 1 entspricht, wird ein weiteres vorteilhaftes Detail des erfindungsgemäßen Inkontinenzartikels erläutert. Durch die Verfolgung des dreikomponentigen Konzepts zur Herstellung des erfindungsgemäßen Inkontinenzartikels ergibt sich ein Übergang 90 zwischen Schrittabschnitt 8 und Bauchabschnitt 4 sowie ein Übergang 92 zwischen Schrittabschnitt 8 und Rückenabschnitt 6, bei denen sich üblicherweise ein unstetiger, also mit Ecken oder Winkeln oder Knicken versehener Verlauf der die Beinöffnungen 19 begrenzenden Ränder der Chassismaterialien ergibt. Dies birgt die Gefahr, dass im Bereich der Übergänge 90, 92 Kraftspitzen gebildet werden, die zu einem Einreißen der Chassismaterialien führen können, was die Anfügung des Schrittabschnitts 8 an den Bauchschnitt 4 beziehungsweise an den Rückenabschnitt 6 beeinträchtigen kann. Um dem entgegen zu wirken ist im jeweiligen Übergang 90 und 92 eine verstärkende Beschichtung 94, 96 des flüssigkeitsundurchlässigen Backsheet-Materials 62 des Schrittabschnitts 8 vorgesehen. Es erweist sich als hinreichend, wenn diese verstärkende Beschichtung 94, 96 nur in dem durch die unterbrochene Linie jeweils bezeichneten Bereich der Figur 7 vorgesehen wird. Die verstärkende Beschichtung 94, 96 überlappt im beispielhaft und vorzugsweise dargestellten Fall den Bauchabschnitt 4 und den Rückenabschnitt 6 in Längsrichtung 9 lediglich um etwa 10 bis 20 mm, insbesondere um ca. 15 mm. Die verstärkende Beschichtung endet in Längsrichtung 9 jeweils vor den Längsenden 98, 100 des Schrittabschnitts, und zwar wenigstens 30 mm vor dem bauchseitigen Längsende 98 und wenigstens 90 mm vor dem rückenseitigen Längsende 100. Dies erweist sich als vorteilhaft, da solchenfalls die verstärkende Beschichtung 94, 96 nicht unnötig zur Versteifung der Chassismaterialien in Bereichen beiträgt, wo dies nicht hilfreich, sondern eher unerwünscht und nachteilig ist. Außerdem können auf diese Weise Materialkosten gespart werden. Jedoch bleibt die Möglichkeit unberührt, die verstärkende Beschichtung 94, 96 nicht nur im Übergang 90 beziehungsweise 92 vorzusehen.

Die verstärkende Beschichtung 94, 96 besteht vorzugsweise aus einem Vliesstoff, insbesondere einem Spinnvlies aus Polypropylen, insbesondere mit einem Flächengewicht von 10 - 20 g/m$^2$, insbesondere von 12 - 17 g/m$^2$.

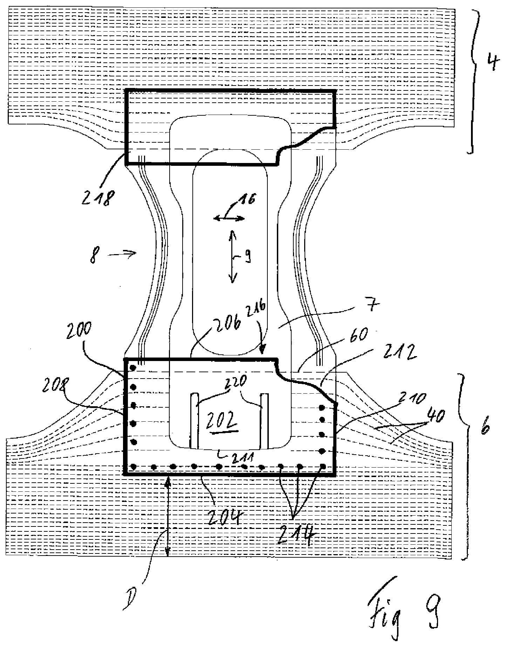

Schließlich verdeutlicht Figur 9 die erfindungsgemäße Ausbildung des Inkontinenzartikels, wobei alle vorausgehend im Zusammenhang mit den Figuren 1-8 dargestellten oder erörterten Komponenten, Merkmale und Einzelheiten einzeln und in beliebiger Kombination im Zusammenwirken mit den neuen nachfolgend zu beschreibenden Komponenten der Figur 9 als vorteilhaft und zur Erfindung gehörig angesehen werden. Dargestellt ist ein Flachmaterialabschnitt 200, der einen hinteren dem Rückenabschnitt 6 zugewandten Endbereich 202 des Absorptionskörpers 7 des Schrittabschnitts 8 in Längsrichtung 9 und in Querrichtung 16 überfängt. Der Flachmaterialabschnitt 200 umfasst einen hüftzugewandten im wesentlichen in der Querrichtung 16 verlaufenden Rand 204 und einen schrittzugewandten, im wesentlichen in der Querrichtung 16 verlaufenden Rand 206 sowie zwei Seitenränder 208, 210. Im dargestellten Fall erstreckt sich der schrittzugewandte Rand 206 des Flachmaterialabschnitts 200 über den schrittzugewandten Rand 60 des Rückenabschnitts 6 hinaus. Nach einer alternativen Ausführungsform wäre es auch denkbar, den Flachmaterialabschnitt 200 in der Längsrichtung 9 betrachtet kürzer auszubilden. Solchenfalls würde sich der schrittzugewandte Rand 206 des Flachmaterialabschnitts 200 vorzugsweise lediglich über den hüftzugewandten Rand 211 des Absorptionskörpers 7 hinaus erstrecken, nicht aber über den schrittzugewandten Rand 60 des Rückenabschnitts 6. Vorzugsweise würde sich der schrittzugewandte Rand 206 des Flachmaterialabschnitts 200 gleichwohl bis in den schrittseitigen und den Beinöffnungen zugewandten Bereich 26 (siehe Figur 1) des Rückenabschnitts 6 erstrecken.

Der hüftzugewandte Rand 204 des Flachmaterialabschnitts 200 weist in Längsrichtung 9 einen Abstand D vom benachbarten Längsende des Rückenabschnitts 6 auf. Abstand D beträgt im beispielhaft dargestellten Fall 12 cm.

Der Flachmaterialabschnitt 200 ist auf die körperzugewandte Seite 212 des Schrittabschnitts 8 fixiert und zwar im rein beispielhaft dargestellten Fall durch eine Abfolge von Ultraschweißpunkten 214, die sich entlang der drei Ränder 204, 208, 210, nicht aber entlang des schrittzugewandten Rands 206 erstrecken. Der schrittzugewandte Rand 206 ist elastisch oder vorzugsweise elastifiziert ausgebildet, indem dort in einem beispielsweise umgeschlagenen Randbereich ein insbesondere fadenförmiges Elastifizierungsmittel vorgesehen ist. Die Elastifizierung dieses schrittzugewandten Rands 206 des Flachmaterialabschnitts 200 könnte aber auch in anderer auf dem hier in Rede stehenden Gebiet üblichen Weise realisiert sein. Durch diese Elastifizierung vermag sich der Flachmaterialabschnitt von der körperzugewandten Oberseite 212 des Schrittabschnitts 8 abzuheben, so dass eine in der Längsrichtung 9 öffnende Tasche 216 gebildet wird, welche Körperausscheidungen aufnehmen kann und ein Fortschreiten von Körperausscheidungen in Richtung auf den Rückenabschnitt 6 verhindern kann.

Im beispielhaft dargestellten Fall ist auch am bauchseitigen Ende des Schrittabschnitts 8 ein vorzugsweise entsprechend ausgebildeter Flachmaterialabschnitt 218 vorgesehen.

Dadurch, dass unterhalb des Absorptionskörpers 7 die zweiten Elastifizierungsmittel 40 nicht vorgesehen oder durch an sich bekannte Maßnahmen ihrer elastifizierenden Wirkung benommen sind, wird das Abheben des Flachmaterialabschnitts 200 von der Oberseite des Schrittabschnitts 8 im Bereich des schrittseitigen Rands 206 und damit das Öffnen der Tasche 216 begünstigt.

Nach einem weiteren erfindungsgemäßen Vorschlag ist in dem Absorptionskörper 7, und zwar zumindest in seinem rückenseitigen Endbereich 202, wenigstens eine, im beispielhaft dargestellten Fall zwei Flexibilitäts- oder Sollbiegeachsen 220 ausgebildet, die in der Tragesituation des Inkontinenzartikels das Abheben des schrittseitigen Rands 206 des Flachmaterialabschnitts 200 durch eine nach oben konkave Krümmung des Absorptionskörpers 7 bzw. des Schrittabschnitts 8 begünstigen. Die Anzahl, Ausbildung und Position dieser beispielhaft dargestellten Flexibilitäts-oder Sollbiegeachsen 220 ist nicht limitierend, sondern eine entsprechende nach oben konkav öffnende Gestalt des Absorptionskörpers 7 bzw. Schrittabschnitts 8 kann auch durch anders gestaltete oder angeordnete Achsen erreicht werden.

## Patentansprüche

1. Inkontinenzartikel (2) in Höschenform für die Aufnahme von Körperausscheidungen mit einem vorderen Bauchabschnitt (4) und einem hinteren Rückenabschnitt (6), die in einer Längsrichtung (9) des Inkontinenzartikels voneinander separat und beabstandet sind, und mit einem einen Absorptionskörper (7) aufweisenden Schrittabschnitt (8), der sich in der Längsrichtung (9) zwischen dem Bauchabschnitt (4) und dem Rückenabschnitt (6) erstreckt und an den Bauchabschnitt (4) und an den Rückenabschnitt (6) unlösbar angefügt ist, wobei der Bauchabschnitt (4) und der Rückenabschnitt (6) zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind und wobei sowohl der Schrittabschnitt (8) als auch der Bauchabschnitt (4) und der Rückenabschnitt (6) Beinöffnungen (19) des Inkontinenzartikels begrenzen, wobei in einem hüftseitigen Bereich (20, 24) des Bauchabschnitts (4) und des Rückenabschnitts (6) erste Elastifizierungsmittel (28) vorgesehen sind, die sich in einem Abstand voneinander und parallel zueinander in Quer- oder Hüftumfangsrichtung (16) erstrecken und so den Bauchabschnitt (4) und den Rückenabschnitt (6) in dem genannten hüftseitigen Bereich (20, 24) über dessen gesamte flächenhafte Erstreckung durchgehend elastifizieren, wobei in einem schrittseitigen und den Beinöffnungen (19) zugewandten Bereich (22, 26) des Rückenabschnitts (6) und vorzugsweise auch des Bauchabschnitts (4) zweite Elastifizierungsmittel (40) vorgesehen sind, die sich ausgehend von den beiden Seitennahtbereichen (14) in Richtung (56) auf eine Längsmittelachse (44) des Inkontinenzartikels erstrecken, wobei die zweiten Elastifizierungsmittel (40) im Überlappungsbereich des Absorptionskörpers (7) mit dem Bauchabschnitt (4) und mit dem Rückenabschnitt (6) entweder nicht vorhanden oder ihrer elastifizierenden Wirkung benommen sind, **dadurch gekennzeichnet, dass** ein hinterer dem Rückenabschnitt (6) zugewandter Endbereich (202) des Absorptionskörpers (7) in Längsrichtung (9) und in Querrichtung (16) von einem Flachmaterialabschnitt (200) überfangen ist, der auf der körperzugewandten Seite (212) des Schrittabschnitts (8) vorgesehen ist und an den Schrittabschnitt (8) unlösbar angefügt ist, und dass der Flachmaterialabschnitt (200) einen hüftzugewandten im wesentlichen in Querrichtung (16) verlaufenden Rand (204) und einen schrittzugewandten im wesentlichen in Querrichtung (16) verlaufenden Rand (206) und zwei Seitenränder (208, 210) aufweist und dass der hüftzugewandte Rand (204) in Längsrichtung (9) einen Abstand vom benachbarten Längsende des Rückenabschnitts (6) aufweist, und dass der Flachmaterialabschnitt (200) im Bereich seines schrittzugewandten Rands (206) oberhalb des Absorptionskörpers (7) nicht gegen den Schrittabschnitt (8) fixiert ist, so dass er sich von dem darunter liegenden Schrittabschnitt (8) erheben und eine in Längsrichtung (9) öffnende Tasche (216) für Körperausscheidungen bilden kann, und dass der schrittzugewandte Rand (206) des Flachmaterialabschnitts (200) elastisch oder elastifiziert ausgebildet ist und dass der Flachmaterialabschnitt (200) mit seinem hüftzugewandten im wesentlichen in Querrichtung (16) verlaufenden Rand (204) und mit seinen beiden Seitenrändern (208, 210) bis wenigstens nahezu an die Ränder des Schrittabschnitts (8) heranreicht, also nicht über die Peripherie des Schrittabschnitts (8) übersteht, und dass der Flachmaterialabschnitt (200) entlang seines hüftzugewandten im wesentlichen in Querrichtung (16) verlaufenden Rands (204) und entlang seiner beiden Seitenränder (208, 210) kontinuierlich oder diskontinuierlich gegen die körperzugewandte Seite (212) des Schrittabschnitts (8) fixiert ist.

2. Inkontinenzartikel nach Anspruch 1, **dadurch gekennzeichnet, dass** der schrittzugewandte Rand (206) des Flachmaterialabschnitts (200) über seine gesamte Länge elastisch oder elastifiziert ausgebildet ist.

3. Inkontinenzartikel nach einem oder mehreren der vorstehenden Ansprüche, **dadurch gekennzeichnet, dass** der Absorptionskörper (7) in seinem hinteren dem Rückenabschnitt (6) zugewandten Endbereich (202) wenigstens eine ungefähr in Längsrichtung (9) erstreckte Flexibilitäts- oder Sollbiegeachse (220) aufweist, was die Bildung der in Längsrichtung (9) öffnenden Tasche (216) begünstigt.

4. Inkontinenzartikel nach Anspruch 3, **dadurch gekennzeichnet, dass** die Flexibilitäts- oder Sollbiegeachse (220) am hüftseitigen Ende des Absorptionskörpers (7) ausmündet.

5. Inkontinenzartikel nach einem oder mehreren der vorstehenden Ansprüche, **dadurch gekennzeichnet, dass** die Erstreckung des Flachmaterialabschnitts (200) von seinem schrittzugewandten Rand (206) in Längsrichtung (9) nach hinten bis zu einer Fixierung im Bereich des hüftzugewandten im wesentlichen in Querrichtung (16) verlaufenden Rands (204) wenigstens 2 cm und weiter insbesondere höchstens 15 cm beträgt.

6. Inkontinenzartikel nach einem oder mehreren der vorstehenden Ansprüche, **dadurch gekennzeichnet, dass** die zweiten Elastifizierungsmittel (40), die sich in dem schrittseitigen Bereich (22, 26) des Rückenabschnitts (6) und vorzugsweise auch des Bauchabschnitts (4) ausgehend von den beiden Seitennahtbereichen (14) in Richtung (56) auf eine Längsmittelachse (44) des Inkontinenzartikels erstrecken, dabei bogenförmig verlaufen und sich in Richtung auf die Längsmittelachse (44) auffächern, so dass ihr Abstand voneinander zunimmt.

7. Inkontinenzartikel nach einem oder mehreren der vorstehenden Ansprüche, **dadurch gekennzeichnet, dass** ein vorderer dem Bauchabschnitt (4) zugewandter Endbereich des Absorptionskörpers (7) in Längsrichtung (9) und in Querrichtung (16) von einem Flachmaterialabschnitt (218) überfangen ist, der auf der körperzugewandten Seite des Schrittabschnitts (8) vorgesehen ist und an den Schrittabschnitt (8) und/oder an den Bauchabschnitt (4) unlösbar angefügt ist, und dass der Flachmaterialabschnitt (218) einen hüftzugewandten im wesentlichen in Querrichtung verlaufenden Rand und einen schrittzugewandten im wesentlichen in Querrichtung verlaufenden Rand und zwei Seitenränder aufweist und dass der hüftzugewandte Rand in Längsrichtung einen Abstand vom benachbarten Längsende des Bauchabschnitts (4) aufweist, und dass der Flachmaterialabschnitt (218) im Bereich seines schrittzugewandten Rands oberhalb des Absorptionskörpers (7) nicht gegen den Schrittabschnitt (8) fixiert ist, so dass er sich von dem darunter liegenden Schrittabschnitt (8) erheben und eine in Längsrichtung (9) öffnende Tasche für Körperausscheidungen bilden kann, und dass der schrittzugewandte Rand des Flachmaterialabschnitts (218) elastisch oder elastifiziert ausgebildet ist.

## Claims

1.  An incontinence article (2) in panty-type for holding bodily excretions, comprising a front stomach portion (4) and a rear back portion (6), which are separate from each other and spaced apart from each other in a longitudinal direction (9) of the incontinence article, and comprising a crotch portion (8), which has an absorption body (7) and extends in the longitudinal direction (9) between the stomach portion (4) and the back portion (6) and is joined inseparably to the stomach portion (4) and to the back portion (6), wherein the stomach portion (4) and the back portion (6) are connected to each other at side-seam areas (14) on both sides by the manufacturer to form an abdominal and back band, which is continuous in a transverse or hip peripheral direction (16) and has a hip opening (18), which is closed in the hip peripheral direction, and wherein the crotch portion (8), the stomach portion (4), and the back portion (6) delimit leg openings (19) of the incontinence article, wherein first elasticating means (28) are provided in a waist-side area (20, 24) of the stomach portion (4) and of the back portion (6) and extend at a distance from each other and parallel to each other in the transverse or hip peripheral direction (16) and thus continuously provide elasticity to the stomach portion (4) and the back portion (6) in said waist-side area (20, 24) across the entire surface extent thereof, wherein second elasticating means (40) are provided in a crotch-side area (22, 26) of the back portion (6) and preferably also of the stomach portion (4) that faces the leg openings (19) and extend from the two side-seam areas (14) in the direction (56) toward a longitudinal centre axis (44) of the incontinence article, wherein the second elasticating means (40), in the area of overlap of the absorption body (7) with the stomach portion (4) and the back portion (6), are either not present or are deprived of their elasticity action **characterized in that** a rear end area (202) of the absorption body (7) facing the back portion (6) is covered in the longitudinal direction (9) and in the transverse direction (16) by a flat-material section (200), which is provided on the body-facing side (212) of the crotch portion (8) and is joined inseparably to the crotch portion (8), and that the flat-material section (200) has a hip-facing edge (204) extending substantially in the transverse direction (16) and a crotch-facing edge (206) extending substantially in the transverse direction (16) and two side edges (208, 210) and that the hip-facing edge (204) has a distance from the adjacent longitudinal end of the back portion (6) in the longitudinal direction (9), and that the flat-material section (200) is not fastened to the crotch portion (8) in the area of the crotch-facing edge (206) of the flat-material section (200) above the absorption body (7) so that the flat-material section (200) can rise from the crotch portion (8) lying thereunder and can form a pocket (216) that opens in the longitudinal direction (9), and that the crotch-facing edge (206) of the flat-material section (200) is elastic or provided with elasticity, and that the flat-material section (200) at least nearly reaches the edges of the crotch portion (8) by means of the hip-facing edge (204) of the flat-material section (200) extending substantially in the transverse direction (16) and by means of the two side edges (208, 210) of the flat-material section (200), i.e., does not protrude beyond the periphery of the crotch portion (8), and that the flat-material section (200) is fastened to the body-facing side (212) of the crotch portion (8) continuously or discontinuously along the hip-facing edge (204) of the flat-material section (200) extending substantially in the transverse direction (16) and along the two side edges (208, 210) of the flat-material section (200).

2.  The incontinence article according to claim 1, **characterized in that** the crotch-facing edge (206) of the flat-material section (200) is elastic or provided with elasticity over the entire length thereof.

3.  The incontinence article according to one or more of the preceding claims, **characterized in that** the absorption body (7) has, in the rear end area (202) thereof facing the back portion (6), at least one flexibility or desired bending axis (220) extending approximately in the longitudinal direction (9), the formation of the pocket (216) that opens in the longitudinal direction (9) thus being promoted.

4.  The incontinence article according to claim 3, **characterized in that** the flexibility or desired bending axis (220) ends out at the hip-side end of the absorption body (7).

5.  The incontinence article according to one or more of the preceding claims, **characterized in that** the extent of the flat-material section (200) from the crotch-facing edge (206) thereof rearward in the longitudinal direction (9) to a fastening connection in the area of the hip-facing edge (204) extending substantially in the transverse direction (16) is at least 2 cm and, furthermore, in particular at most 15 cm.

6. The incontinence article according to one or more of the preceding claims, **characterized in that** the second elasticating means (40), which extend in the crotch-side area (22, 26) of the back portion (6) and preferably also of the stomach portion (4) from the two side-seam areas (14) in the direction (56) toward a longitudinal centre axis (44) of the incontinence article, thereby extend in an arcuate manner and fan out in the direction toward the longitudinal centre axis (44) so that the distance of the second elasticating means (40) from each other increases.

7. The incontinence article according to one or more of the preceding claims, **characterized in that** a front end area of the absorption body (7) facing the stomach portion (4) is covered in the longitudinal direction (9) and in the transverse direction (16) by a flat material section (218), which is provided on the body-facing side (212) of the crotch portion (8) and is joined inseparably to the crotch portion (8) and/or to the stomach portion (4), and that the flat material section (218) has a hip-facing edge extending substantially in the transverse direction and a crotch-facing edge extending substantially in the transverse direction and two side edges and that the hip-facing edge has a distance from the adjacent longitudinal end of the stomach portion (4) in the longitudinal direction, and that the flat material section (218) is not fastened to the crotch portion (8) in the area of the crotch-facing edge of the flat material section (218) above the absorption body (7) so that the flat material section (218) can rise from the crotch portion (8) lying thereunder and can form a pocket for bodily excretions that opens in the longitudinal direction (9), and that the crotch-facing edge of the flat material section (218) is elastic or provided with elasticity.

**Revendications**

1. Article d'incontinence (2) sous forme de culotte, destiné à recueillir les excrétions corporelles, comprenant une portion ventrale avant (4) et une portion dorsale arrière (6) qui sont séparées et espacées l'une de l'autre dans une direction longitudinale (9) de l'article d'incontinence, et comprenant une portion d'entrejambe (8) présentant un corps absorbant (7), qui s'étend dans la direction longitudinale (9) entre la portion ventrale (4) et la portion dorsale (6) et qui est rapportée de manière inamovible sur la portion ventrale (4) et sur la portion dorsale (6), dans lequel, pour la formation d'une bande ventrale et dorsale continue dans le sens transversal ou dans le sens du tour de hanches (16), avec une ouverture de hanche (18) fermée dans le sens du tour de hanches, ladite portion ventrale (4) et ladite portion dorsale (6) sont reliées entre elles par le fabricant au niveau de zones de couture latérale (14) situées de part et d'autre, et dans lequel aussi bien ladite portion d'entrejambe (8) que la portion ventrale (4) et la portion dorsale (6) délimitent des ouvertures de jambe (19) de l'article d'incontinence, dans lequel de premiers moyens d'élastification (28) sont prévus dans une zone côté hanche (20, 24) de la portion ventrale (4) et de la portion dorsale (6), qui s'étendent à une distance les uns des autres et parallèlement entre eux dans le sens transversal ou dans le sens du tour de hanches (16) et qui élastifient ainsi de manière continue lesdites portions ventrale (4) et dorsale (6) dans ladite zone côté hanche (20, 24) sur l'ensemble de son extension en nappe, dans lequel de seconds moyens d'élastification (40) sont prévus dans une zone (22, 26) de la portion dorsale (6) et de préférence également de la portion ventrale (4), qui est située côté entrejambe et montre vers les ouvertures de jambe (19), seconds moyens d'élastification qui s'étendent à partir des deux zones de couture latérale (14) en direction (56) d'un axe médian longitudinal (44) de l'article d'incontinence, dans lequel les seconds moyens d'élastification (40) soit n'existent pas dans la zone de chevauchement du corps absorbant (7) avec la portion ventrale (4) et avec la portion dorsale (6) soit y sont dépourvus de leur effet élastifiant, **caractérisé par le fait qu'**une zone d'extrémité (202) arrière du corps absorbant (7), qui montre vers la portion dorsale (6), est revêtue, dans la direction longitudinale (9) et dans la direction transversale (16), d'une portion en matière plate (200) qui est prévue sur la face (212) de la portion d'entrejambe (8), laquelle montre vers le corps, et qui est rapportée de manière inamovible sur la portion d'entrejambe (8), et que la portion en matière plate (200) présente un bord (204) montrant vers la hanche et s'étendant pour l'essentiel dans la direction transversale (16) ainsi qu'un bord (206) montrant vers l'entrejambe et s'étendant pour l'essentiel dans la direction transversale et deux bords latéraux (208, 210), et que le bord (204) montrant vers la hanche présente, dans la direction longitudinale (9), une distance de l'extrémité longitudinale voisine de la portion dorsale (6), et que, au niveau de son bord (206) montrant vers l'entrejambe, au-dessus du corps absorbant (7), la portion en matière plate (200) n'est pas fixée contre la portion d'entrejambe (8) de sorte qu'elle peut se détacher de la portion d'entrejambe (8) située dessous et peut former une poche (216) pour des excrétions corporelles qui s'ouvre dans la direction longitudinale (9), et que le bord montrant vers l'entrejambe (206) de la portion en matière plate (200) est réalisé de manière élastique ou élastifiée et que la portion en matière plate (200) s'étend, par son bord (204) montrant vers la hanche et s'étendant pour l'essentiel dans la direction transversale (16) et par ses deux bords latéraux (208, 210), au moins approximativement jusqu'aux bords de la portion d'entrejambe (8), donc ne dépasse pas par ceux-ci la périphérie de la portion d'entrejambe (8), et que la portion en matière plate (200) est fixée de manière continue ou discontinue contre la face (212) de la portion d'entrejambe (8), qui montre vers le corps, le long de son bord (204) montrant vers la hanche et s'étendant pour l'essentiel dans la direction transversale (16) et le long de ses deux bords latéraux (208, 210).

2. Article d'incontinence selon la revendication 1, **caractérisé par le fait que** le bord montrant vers l'entrejambe (206) de la portion en matière plate (200) est réalisé de manière élastique ou élastifiée sur l'ensemble de sa longueur.

3. Article d'incontinence selon l'une ou plusieurs des revendications précédentes, **caractérisé par le fait que** le corps absorbant (7) présente, dans sa zone d'extrémité (202) arrière montrant vers la portion dorsale (6), au moins un axe de flexibilité ou destiné à la flexion (220) s'étendant à peu près dans la direction longitudinale (9), ce qui favorise la formation de ladite poche (216) s'ouvrant dans la direction longitudinale (9).

4. Article d'incontinence selon la revendication 3, **caractérisé par le fait que** ledit axe de flexibilité ou destiné à la flexion (220) débouche à l'extrémité côté hanche du corps absorbant (7).

5. Article d'incontinence selon l'une ou plusieurs des revendications précédentes, **caractérisé par le fait que** l'extension de la portion en matière plate (200) depuis son bord (206) montrant vers l'entrejambe, dans la direction longitudinale (9), en arrière jusqu'à son fixation au niveau du bord (204) montrant vers la hanche et s'étendant pour l'essentiel dans la direction transversale (16) est de 2 cm au moins et plus particulièrement de 15 cm tout au plus.

6. Article d'incontinence selon l'une ou plusieurs des revendications précédentes, **caractérisé par le fait que** les seconds moyens d'élastification (40) qui s'étendent dans la zone côté entrejambe (22, 26) de la portion dorsale (6) et de préférence également de la portion ventrale (4), à partir des deux zones de couture latérale (14) en direction (56) d'un axe médian longitudinal (44) de l'article d'incontinence, s'étendent en arc ainsi qu'en éventail en direction de l'axe médian longitudinal (44) de sorte que leur distance les uns des autres augmente.

7. Article d'incontinence selon l'une ou plusieurs des revendications précédentes, **caractérisé par le fait qu'**une zone d'extrémité avant du corps absorbant (7), qui montre vers la portion ventrale (4), est revêtue, dans la direction longitudinale (9) et dans la direction transversale (16), d'une portion en matière plate (218) qui est prévue sur la face de la portion d'entrejambe (8), montrant vers le corps, et qui est rapportée de manière inamovible sur la portion d'entrejambe (8) et/ou sur la portion ventrale (4), et que la portion en matière plate (218) présente un bord montrant vers la hanche et s'étendant pour l'essentiel dans la direction transversale ainsi qu'un bord montrant vers l'entrejambe et s'étendant pour l'essentiel dans la direction transversale et deux bords latéraux, et que le bord montrant vers la hanche présente, dans la direction longitudinale, une distance de l'extrémité longitudinale voisine de la portion ventrale (4), et que, au niveau de son bord montrant vers l'entrejambe, au-dessus du corps absorbant (7), ladite portion en matière plate (218) n'est pas fixée contre la portion d'entrejambe (8) de sorte qu'elle peut se détacher de la portion d'entrejambe (8) située dessous et peut former une poche pour des excrétions corporelles qui s'ouvre dans la direction longitudinale (9), et que le bord de la portion en matière plate (218), qui montre vers l'entrejambe, est réalisé de manière élastique ou élastifiée.

EP 2 750 648 B1

Fig. 1

EP 2 750 648 B1

Fig. 2

Fig. 3

14

Fig. 4

Fig. 5

Fig. 6

EP 2 750 648 B1

Fig. 7

16

Fig. 8

Fig 9

**IN DER BESCHREIBUNG AUFGEFÜHRTE DOKUMENTE**

**In der Beschreibung aufgeführte Patentdokumente**

- DE 102007055524 A1

- US 5558660 A